# EUROPÄISCHE PATENTSCHRIFT

(11) **EP 2 386 288 B1**
(45) Veröffentlichungstag und Bekanntmachung des Hinweises auf die Patenterteilung: **09.08.2017**
(21) Anmeldenummer: 10178681.2
(22) Anmeldetag: 23.09.2010
(51) Int. Cl.: A61K 8/37, A61K 8/44, A61K 8/49, A61K 8/65, A61Q 5/10

(54) **Pflegende Haarbehandlungsmittel zur Farbveränderung**
Protective hair treatment agent for dying hair
Produit de soin pour le traitement des cheveux destiné à la coloration

(30) Priorität: 14.12.2009 DE 102009054600
(43) Veröffentlichungstag der Anmeldung: 16.11.2011
(73) Patentinhaber: Henkel AG & Co. KGaA, 40589 Düsseldorf (DE)
(72) Erfinder: Schwartz, Stephan, 22880, Wedel (DE); Ludwig, Meike, 21244, Buchholz i.d. Nordheide (DE); Rippe, Maureen, 29640, Schneeverdingen (DE); Kleen, Astrid, 20457, Hamburg (DE); Akram, Mustafa, 22455, Hamburg (DE)

(56) Entgegenhaltungen:
- WO-A1-97/01323
- DE-A1-102005 017 056
- DE-A1-102005 029 534

## Beschreibung

Die Erfindung betrifft ein Färbemittel für keratinhaltige Fasern für glänzende und pflegende Färbungen. Dieses Färbemittel enthält in einem kosmetischen Träger neben einer farbgebenden Komponente mindestens einen Wirkstoffkomplex enthält, welcher einen bestimmten Diester (a) der Formel (I), ein Proteinhydrolysat aus Collagen aus "*Pisces*" (b) mit einem hohen Anteil von Hydroxyprolin, und L-Serin umfasst. Besonders vorteilhaft erweist sich der Zusatz dieses Wirkstoffkomplexes in oxidativen Färbemitteln. Darüber hinaus betrifft die Erfindung die Verwendung des besagten Mittels zur Verbesserung des Feuchtigkeitsgehalts und des Pflegezustands, insbesondere von Glanz und Naßkämmbarkeit, von keratinischen Fasern, insbesondere menschlichen Haaren, bei der Färbung.

Die Veränderung von Form und Farbe der Haare stellt einen wichtigen Bereich der modernen Kosmetik dar. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Zur Bereitstellung farbverändernder kosmetischer Mittel, insbesondere für die Haut oder keratinhaltige Fasern, wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen.

Als Aufhellmittel kommen insbesondere oxidative Bleich- oder Blondiermittel zum Einsatz. Um beim Bleichvorgang große Farbunterschiede zu erzielen, also insbesondere dunkle oder schwarze Haare zu entfärben, werden neben den üblichen Wasserstoffperoxidzubereitungen zumeist als Aufhellmittel Bleichaktivatoren wie beispielsweise Persalze als zusätzliche Peroxidquellen, und/oder Carbonate als zusätzliche Alkalisierungsmittel benötigt.

Insbesondere oxidative Haarfärbemittel sind trotz ihrer vorteilhaften Färbeeigenschaften für den Anwender mit Nachteilen behaftet. So führt der Einsatz der Oxidationsmittel zur Ausfärbung beziehungsweise Entwicklung der eigentlichen Färbung zu Schädigungen in der Haarstruktur und auf der Haaroberfläche. Das Haar wird brüchig, seine Elastizität lässt nach und die Kämmbarkeit nimmt ab. Zweitens benötigen oxidative Färbemittel in der Regel einen basischen pH-Wert zur Ausfärbung, insbesondere zwischen pH 9,0 und pH 11,5. Das basische Milieu stellt jedoch einen weiteren Grund der Schädigung für das Haar und dessen Struktur dar, der ebenfalls mit gesteigerter Anwendungszeit an Bedeutung gewinnt. Die Spreizung der äußeren Schuppenschicht führt außerdem zu einem unangenehmen Oberflächenempfinden der Haare und damit zu einer verschlechterten Kämmbarkeit im Nass- und Trockenzustand. Dadurch besteht für den Verbraucher eine gesteigerte Notwendigkeit, zusätzliche Nachbehandlungsmittel einsetzen.

Um den Pflegezustand der Fasern zu verbessern, ist es seit langem üblich, die Fasern im Anschluss an die farbverändernde Behandlung einer speziellen Nachbehandlung zu unterziehen. Um den Aufwand der üblichen mehrstufigen Verfahren, insbesondere bei der direkten Anwendung durch Verbraucher, zu verringern, wurden in jüngster Zeit sogenannte Kombinationspräparate entwickelt. Diese Präparate enthalten neben den üblichen Komponenten, beispielsweise zur Färbung der Haare, zusätzlich Wirkstoffe, die früher den Haarnachbehandlungsmitteln vorbehalten waren. Um den zusätzlichen Nachbehandlungsschritt einzusparen, hat es nicht daher an Versuchen gemangelt, geeignete Pflegestoffe in die Haarfärbemittel einzuarbeiten. Üblicherweise werden oxidative Haarfärbemittel unmittelbar vor der Anwendung aus einer Färbezubereitung und einer sogenannten Entwicklerzubereitung hergestellt. In der Regel besitzt die Färbezubereitung einen stark basischen pH-Wert, um die darin enthaltenen Oxidationsfarbstoffvorprodukte zu stabilisieren, während die Entwicklerzubereitung einen schwach sauren pH-Wert, dafür aber die zur Farbstoffbildung notwendigen Oxidationsmittel enthält. Beide Zubereitungen stellen somit kein vorteilhaftes Umfeld dar, einen chemisch empfindlichen Pflegestoff ohne Zersetzung zu beinhalten. Es besteht daher weiterhin ein Bedarf an geeigneten, stabilen Pflegestoffen, die sich in oxidative Färbemittel einarbeiten lassen und so bereits während des Färbevorgangs auftretende Schädigungen zu minimieren vermögen.

Aufgabe der vorliegenden Erfindung ist es daher, die oben genannten Nachteile oxidativer Haarfärbemittel herabzusenken. Die Färbemittel sollen das Haar schützen und damit eine verringerte Schädigung des Haares bewirken. Insbesondere soll durch die Mittel der Feuchtigkeitsgehalt in der Faser verbessert werden, was dem Haar erhöhte Elastizität und Geschmeidigkeit sowie einen verbesserten Glanz verleiht. Die Verringerung von Haarschädigungen während der Färbung soll jedoch nicht zu Lasten einer verringerten Färbeleistung der Mittel erreicht werden. DE 10 2005 029 534 offenbart Haarfärbemitteln, die Kämmbarkeit und Glanz der Haare verbessern und dafür Proteinhydrolysate und/oder Aminosäure als Pflegestoffe verwenden. Bevorzugte Proteinhydrolysate sind beispielsweise Collagenhydrolysate von Fischen. Es wurde nun in nicht vorhersehbarer Weise gefunden, dass der Zusatz von einem Wirkstoffkomplex, welcher einen bestimmten Diester (a) der Formel (I) sowie ein Proteinhydrolysat aus Collagen aus "*Pisces*" (b) mit einem hohen Anteil von Hydroxyprolin, und L-Serin in bestimmten Verhältnissen zueinander umfasst, in Färbemitteln für keratinische Fasern zu Vorteilen gegenüber herkömmlichen Färbemitteln hinsichtlich Glanz und Pflege der Fasern führt.

Ein erster Gegenstand der Erfindung ist daher ein Mittel zur Farbveränderung von keratinischen Fasern, insbesondere menschlichen Haaren, welches in einem kosmetischen Träger mindestens eine farbverändernde Komponente enthält und dadurch gekennzeichnet ist, dass das Mittel zusätzlich einen Wirkstoffkomplex enthält, welcher
a) mindestens einen Diester (a) der Formel (I), 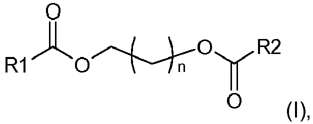 worin
   R1 und R2 jeweils unabhängig voneinander für eine C₇-C₂₃-Alkyl-Gruppe oder C₇-C₂₃-Alkenyl-Gruppe und n für eine Zahl von 1 bis 10 stehen,
b) mindestens ein Proteinhydrolysat (b) mit einem Anteil von Hydroxyprolin von 10 bis 18 Gew.-%, bezogen auf den Proteingesamtgewicht des Proteinhydrolysats, und
c) L-Serin und/oder dessen physiologisch verträglichen Salze,
wobei das Verhältnis von Gesamtgewicht an L-Serin und/oder dessen physiologisch verträglichen Salze zu Proteingesamtgewicht des Proteinhydrolysats b), jeweils bezogen auf das Gesamtgewicht des anwendungsbereiten Mittels, einen Wert von mindestens 1,0 besitzt,
wobei der Wirkstoffkomplex als Proteinhydrolysat mindestens ein Hydrolysat aus Collagen aus "*Pisces*" enthält, umfasst.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die zur erfindungsgemäßen Verwendung eingesetzten Zubereitungen enthalten die Wirkstoffe in einem kosmetischen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Als ersten wesentlichen Inhaltsstoff enthält die Färbezubereitung zur erfindungsgemäßen Verwendung mindestens eine farbverändernde und/oder farbgebende Komponente.

Die farbverändernde Komponente wird dabei ausgewählt aus mindestens einem Oxidationsfarbstoffvorprodukt und/oder einem direktziehenden Farbstoff und/oder einem Aufhellmittel.

In einer bevorzugten Ausführungsform des ersten Erfindungsgegenstands enthält das Mittel als farbgebende Komponente mindestens ein Oxidationsfarbstoffvorprodukt.

Als Oxidationsfarbstoffvorprodukte enthalten die Färbezubereitungen mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Di-aminobenzo-1,4-dioxan sowie ihren physiologisch verträglichen Salzen. Erfindungsgemäß bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1 H-imidazol-1-yl)propyl]amin, 2-Methoxymethyl-p-phenylendiamin sowie deren physiologisch verträglichen Salzen. Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-di-aminophenyl)-1,4,7,10-tetraoxadecan sowie ihre physiologisch verträglichen Salze. Bevorzugte zweikernige Entwicklerkomponenten werden insbesondere ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines deren physiologisch verträglichen Salzen. Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugt sind p-Aminophenol, 4-Amino-3-methylphenol, 4-Amino-2-aminomethylphenol, 4-Amino-2-(1,2-dihydroxyethyl)phenol und 4-Amino-2-(diethylamino-methyl)phenol. Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazino-pyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze, insbesondere jedoch 4,5-Diamino-1-(2-hydroxyethyl)pyrazol. Als Pyrazolopyrimidine sind insbesondere Pyrazolo[1,5-a]-pyrimidine bevorzugt, wobei bevorzugte Pyrazolo[1,5-a]pyrimidine ausgewählt sind aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]-pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]-pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1 H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]-amin, und/oder 4,5-Diamino-1-(2-hydroxyethyl)pyrazol sowie deren physiologisch verträglichen Salze. Die Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Erfindungsgemäße Kupplerkomponenten werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidin-derivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetra-hydrochinoxalin, und/oder Gemischen aus zwei oder mehreren Verbindungen aus einer oder mehreren dieser Klassen.

Die erfindungsgemäß verwendbaren m-Aminophenole bzw. deren Derivate werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Di-hydroxy-5-(methylamino)benzol, 3-Ethylamino-4-methylphenol, 2,4-Dichlor-3-aminophenol und deren physiologisch verträglichen Salzen. Die erfindungsgemäß verwendbaren 3-Diaminobenzole bzw. deren Derivate werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-di-aminophenoxy)propan, 1-Methoxy-2-amino-4-(2'-hydroxyethylamino)benzol, 1,3-Bis(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Die erfindungsgemäß verwendbaren o-Diaminobenzole bzw. deren Derivate werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Die erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Die erfindungsgemäß verwendbaren Indolderivate werden bevorzugt ausgewählt aus 4-Hydroxyindol, 6-Hydroxyindol und 7-Hydroxyindol und deren physiologisch verträglichen Salzen. Die erfindungsgemäß verwendbaren Indolinderivate werden bevorzugt ausgewählt aus 4-Hydroxyindolin, 6-Hydroxyindolin und 7-Hydroxyindolin und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diamino-phenoxy)propan, 1-Methoxy-2-amino-4-(2-hydroxyethylamino)benzol, 1,3-Bis(2,4-diaminophenyl)-propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)-ethanol, 2-({3-[(2-Hydroxyethyl)amino]-4,5-dimethylphenyl}amino)ethanol, 2-[3-Morpholin-4-yl-phenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethyl-pyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Oxidationsfarbstoffvorprodukte vom Entwicklertyp und vom Kupplertyp werden besonders bevorzugt in bestimmten Kombinationen eingesetzt. Mit den als Kombination genannten Oxidationsfarbstoffvorprodukten und/oder deren physiologisch verträglichen Salzen können jedoch auch noch weitere Farbstoffvorprodukte kombiniert werden: p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diamino-phenoxy)propan; p-Toluylendiamin / 1-Methoxy-2-amino-4-(2-hydroxyethylamino)benzol; p-Toluylendiamin / 2-Amino-3-hydroxypyridin;p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis-(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)-ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1 H-imidazol-1-yl)propyl]amin / 5-Amino-2-methylphenol; N-(4-Amino-3-methylphenyl)-N-[3-(1 H-imidazol-1-yl)propyl]amin / 3-Aminophenol; N-(4-Amino-3-methylphenyl)-N-[3-(1H-imidazol-1-yl)-propyl]amin / 2-(2,4-Diaminophenoxy)ethanol; N-(4-Amino-3-methylphenyl)-N-[3-(1H-imidazol-1-yl)-propyl]amin / 1,3-Bis(2,4-diaminophenoxy)propan; N-(4-Amino-3-methylphenyl)-N-[3-(1 H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1 H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)propan; 4,5-Diamino-1-(2-hydroxyethyl)pyrazol / 1-Methoxy-2-amino-4-(2-hydroxyethylamino)benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

Weiterhin können die Mittel zur erfindungsgemäßen Verwendung mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)-amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Zur Steigerung der Aufhellwirkung von Wasserstoffperoxid ist ebenso möglich, als Aufhellmittel sogenannte Bleichkraftverstärker zuzugeben. Hierzu zählen Persulfat-Salze, Siliciumdioxid-Verbindungen sowie insbesondere kationisierte Heterocyclen.

Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die als Bleichkraftverstärker mindestens ein anorganisches Salz, ausgewählt aus Peroxodisulfaten, enthalten. Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Der Einsatz von Persulfatsalzen bzw. Peroxodisulfatsalzen erfolgt in der Regel in Form eines gegebenenfalls entstaubten Pulvers.

Die erfindungsgemäßen wasserfreien Zusammensetzungen anstelle und/oder zusätzlich zu den festen Peroxoverbindungen einen weiteren Bleichkraftverstärker enthalten. Als Bleichkraftverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxo-hexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Leistung der Oxidationsmittelzubereitung kann der erfindungsgemäßen Zusammensetzung daher zusätzlich mindestens eine gegebenenfalls hydratisierte SiO₂-Verbindung zugesetzt werden. Es kann erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder als Wasserglas eingesetzt. Erfindungsgemäß besonders bevorzugt sind Wassergläser. Erfindungsgemäß besonders bevorzugte Wassergläser werden unter den Bezeichnungen Ferrosil 119, Natronwasserglas 40/42, Portil A, Portil AW, Portil W, Portil N und Britesil C20 vertrieben.

Geeignete Bleichkraftverstärker vom Typ kationisierter Heterocyclen sind insbesondere physiologisch verträgliche Salze von Acetyl-1-methylpyridinium, 4-Acetyl-1-methylpyridinium und N-Methyl-3,4-dihydroisochinolinium, besonders bevorzugt 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Die Aufhellmittel sind in dem Mittel in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Als weiteren wesentlichen Inhaltsstoff enthält das erfindungsgemäße Mittel des ersten Erfindungsgegenstands als Pflegestoff zusätzlich einen Wirkstoffkomplex, welcher
a) mindestens einen Diester (a) der Formel (I), 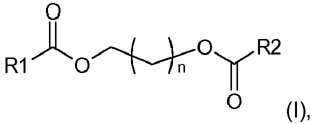 worin
   R1 und R2 jeweils unabhängig voneinander für eine C₇-C₂₃-Alkyl-Gruppe oder C₇-C₂₃-Alkenyl-Gruppe und n für eine Zahl von 1 bis 10 stehen,
b) mindestens ein Proteinhydrolysat (b) mit einem Anteil von Hydroxyprolin von 10 bis 18 Gew.-%, bezogen auf den Proteingesamtgewicht des Proteinhydrolysats, und
c) L-Serin und/oder eines dessen physiologisch verträglichen Salze,
wobei das Verhältnis von Gesamtgewicht an L-Serin und/oder dessen physiologisch verträglichen Salze zu Proteingesamtgewicht des Proteinhydrolysats b), jeweils bezogen auf das Gesamtgewicht des anwendungsbereiten Mittels, einen Wert von mindestens 1,0 besitzt, wobei der Wirkstoffkomplex als Proteinhydrolysat mindestens ein Hydrolysat aus Collagen aus "*Pisces*" enthält, umfasst.

Als Diester der Formel (I) eigenen sich sowohl symmetrische, bei denen R1 und R2 für dieselbe Alkyl- oder Alkenyl-Gruppe stehen, als auch unsymmetrische Diester, bei denen R1 und R2 voneinander unterschiedlich sind. Bevorzugt sind jedoch symmetrische Diester der Formel (I). Bevorzugt steht n für die Zahl 1, 2 oder 3, besonders bevorzugt für die Zahl 1.

Eine Ausführungsform der vorliegenden Erfindung ist dadurch gekennzeichnet, dass das Mittel als Diester der Formel (I) mindestens eine Verbindung enthält, bei der n für die Zahl 1 und R1 und R2 jeweils für eine C₁₇-Alkyl-Gruppe, eine C₁₁-Alkyl-Gruppe oder eine C₁₅-Alkyl-Gruppe stehen. Bevorzugt stehen R1 und R2 für eine C₁₇-Alkyl-Gruppe. Diese Verbindung ist Ethylenglycoldistearat, trägt die INCI-Bezeichnung (Ethylene) Glycol Distearate und wird beispielsweise unter dem Handelsnamen Cutina AGS vertrieben.

Bevorzugt sind in dem erfindungsgemäßen Mittel Diester der Formel (I) in einem Anteil von 0,01 bis 10 Gew.-%, vorzugsweise von 0,05 bis 8 Gew.-% und insbesondere von 0,1 bis 5 Gew.-%, jeweils bezogen auf das Gesamtgewicht des anwendungsbereiten Mittels, enthalten.

Proteinhydrolysate sind bereits als Pflegemittel in der Haarbehandlung bekannt. Sie werden aus unterschiedlichen Quellen gewonnen, wobei sich Proteinhydrolysate mit hohem Gehalt an Hydroxyprolin als besonders vorteilhaft erwiesen haben. Die erfindungsgemäßen Mittel enthalten daher im Wirkstoffkomplex mindestens ein Proteinhydrolysat (b) mit einem Anteil von Hydroxyprolin von 10 bis 18 Gew.-%, bezogen auf den Proteingesamtgewicht des Proteinhydrolysats. Unter Hydroxyprolin dabei ist die Gesamtmenge von 3-Hydroxyprolin und 4-Hydroxyprolin, jeweils in allen diastereomeren und enantiomeren Modifikationen, zu verstehen.

Collagenhydrolysate enthalten dabei überwiegend (2S,4R)-4-Hydroxyprolin und erfüllen die Anforderungen an das Proteinhydrolysat (b) hinsichtlich Hydroxyprolingehalt.

Das zur Hydrolyse eingesetzte Collagen wird überwiegend aus tierischen Bindegeweben gewonnen. Nachteilig an Collagenhydrolysaten ist jedoch die Tatsache, dass sie zumeist aus Gewebe von Säugetieren gewonnen werden. Aufgrund von der Problematik von möglichen Krankheitsübertragungen aus proteinhaltigen Geweben, insbesondere BSE (bovine spongiforme Encephalopathie) und Creutzfeldt-Jakob, ist daher die Suche nach einer alternativen Quelle für Collagenhydrolysate intensiviert worden. Bevorzugt sind daher Collagenhydrolysate aus Collagenen, die nicht aus Säugetieren stammen. Bevorzugt ist dabei ein Collagenhydrolysat aus marinen, nicht-mammalen Lebensformen wie Pisces (Fischen) oder Mollusken. Erfindungsgemäß wird ein Collagenhydrolysat aus *Pisces* (Fischen) verwendet. Eine Ausführungsform des ersten Erfindungsgegenstands ist daher dadurch gekennzeichnet, dass der Wirkstoffkomplex als Proteinhydrolysat mindestens ein Hydrolysat aus Collagen aus "*Pisces*" enthält.

Hierzu können unterschiedliche Collagen-haltigen Gewebe genutzt werden, wie Haut, Knorpel oder Sehnen. Ein Beispiel für ein erfindungsgemäß vorteilhaft einsetzbares Proteinhydrolysat wird aus der Haut von "*Pisces*" (Fischen) gewonnen und unter dem Handelsnamen Marine Hydrolyzed Collagen von der Firma IMPAG vertrieben (INCI-Bezeichnung: Hydrolyzed Collagen).

Bevorzugt sind in dem erfindungsgemäßen Mittel mindestens ein Proteinhydrolysat (b) mit einem Anteil von Hydroxyprolin von 10 bis 18 Gew.-%, bezogen auf den Proteingesamtgewicht des Proteinhydrolysats, mit einem Proteingesamtgehalt von 0,001 bis 1,0 Gew.-%, bevorzugt von 0,005 bis 0,5 Gew.-%, und insbesondere bevorzugt von 0,01 bis 0,1 Gew.-%, jeweils bezogen auf das Gesamtgewicht des anwendungsbereiten Mittels, enthalten.

Weiterhin enthält das erfindungsgemäße Mittel im Wirkstoffkomplex zusätzlich L-Serin und/oder eines deren physiologisch verträglichen Salze.

L-Serin kann dem erfindungsgemäßen Mittel bevorzugt in freier Form zugegeben werden. In einer Reihe von Fällen ist es jedoch auch vorteilhaft, L-Serin in Salzform einzusetzen. Bevorzugte Salze sind dann die Verbindungen mit Halogenwasserstoffsäuren oder Schwefelsäure, insbesondere die Hydrochloride, die Hydrobromide und die Sulfate. Erfindungsgemäß bevorzugt wird dabei L-Serin in freier Form, aber auch als Hydrochlorid eingesetzt.

Um den gewünschten Effekt zu erzielen, hat es sich herausgestellt, dass der Gewichtsanteil an L-Serin größer als das Proteingesamtgewicht des Proteinhydrolysats sein soll.

L-Serin und/oder seine physiologisch verträglichen Salze sind in den erfindungsgemäßen Mitteln bevorzugt in Mengen von 0,01 bis 10 Gew.-%, insbesondere von 0,05 bis 5 Gew.-%, bezogen auf das Gesamtgewicht der Anwendungsmischung, enthalten.

Besonders bevorzugt sind Mittel, bei denen die Komponenten des Wirkstoffkomplexes in ihren Einsatzmengen aufeinander abgestimmt sind.

Eine Ausführungsform des ersten Erfindungsgegenstands ist daher dadurch gekennzeichnet, dass das Mittel einen Wirkstoffkomplex enthält, welcher den oder die Diester (a) der Formel (I) zu 0,01 bis 10,0 Gew.-%, bevorzugt zu 0,1 bis 5,0 Gew.-%, ein Proteinhydrolysat aus Collagen aus *"Pisces"* (b) mit einem Proteingesamtgehalt von 0,001 bis 1,0 Gew.-%, bevorzugt von 0,005 bis 0,2 Gew.-%, und L-Serin und/oder dessen physiologisch verträglichen Salze von 0,05 bis 3,0 Gew.-%, bevorzugt zu 0,1 bis 2,0 Gew.-%, jeweils bezogen auf das Gesamtgewicht des anwendungsbereiten Mittels, enthält.

Eine bevorzugte Ausführungsform des ersten Erfindungsgegenstands ist dadurch gekennzeichnet, dass das anwendungsbereite Mittel zu Farbveränderung als Wirkstoffkomplex eine Kombination aus
(a) 0,1 bis 5,0 Gew.-%, an Ethylenglycoldistearat,
(b) 0,005 bis 0,1 Gew.-% an Proteingesamtgehalt von Marine Hydrolyzed Collagen aus Fischhäuten und
(c) 0,1 bis 2,0 Gew.-% an L-Serin enthält,
wobei sich die Gewichtsanteile jeweils bezogen auf das Gesamtgewicht des anwendungsbereiten Mittels beziehen.

Schließlich hat es sich herausgestellt, dass insbesondere der Zusatz von einem zusätzlichen Pflegestoff, ausgewählt aus kationisierten Phosphatestern, in Mitteln zur Färbung keratinischer Fasern einen besonders vorteilhaften Pflegeeffekt erzeugt.

Eine weitere bevorzugte Ausführungsform der vorliegenden Erfindung ist daher dadurch gekennzeichnet, dass das Mittel zusätzlich einen Pflegestoff, ausgewählt aus kationisierten Phosphatestern, enthält.

Unter einem kationisierten Phosphatester wird im Rahmen der vorliegenden Erfindung ein Tensid der Formel (II) verstanden, 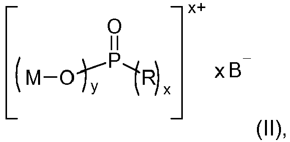 worin
- y: für eine ganze Zahl von 0 bis 2 steht und
- x: für eine ganze Zahl von 1 bis 3 steht, mit der Maßgabe, dass die Summe aus x und y gleich 3 ist;
- M: steht für Wasserstoff, ein Äquivalent eines Alkali- oder Erdalkalimetallkations, ein Ammoniumkation oder einen Alkylrest mit 1 bis 4 Kohlenstoffatomen, der gegebenenfalls mit einer oder mehreren Hydroxygruppe(n) substituiert ist, und
- B: steht für ein Äquivalent eines physiologisch verträglichen Anions, z. B. Chlorid, Bromid, Iodid, Sulfat, Perchlorat, Tetrafluorborat, Tetraphenylborat und Tetrachlorozinkat.

Bevorzugt steht M für ein Natriumkation und B für Chlorid.

R steht in den erfindungsgemäßen Tensiden der Formel (II) für einen Rest der Formel (III), 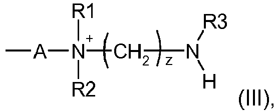 worin z für eine ganze Zahl von 1 bis 4, insbesondere für 3, steht,
- R1, R2: unabhängig voneinander für einen C₁-C₄-Alkylrest stehen, der gegebenenfalls mit einer oder mehreren Hydroxygruppe(n) oder einer Acylgruppe substituiert ist, und
- A: für eine der Einheiten -OCH₂CH₂CH₂-, -OCH₂CH₂- oder -OCH₂CH(OH)CH₂- steht, wobei die Einheit -OCH₂CH(OH)CH₂- erfindungsgemäß besonders bevorzugt ist.
- R3: steht für (a) einen verzweigten oder unverzweigten, gesättigten C₈-C₁₈-Acylrest oder (b) einen verzweigten oder unverzweigten, einfach oder mehrfach ungesättigten C₈-C₁₈-Acylrest.Besonders bevorzugte gesättigte Reste R3 leiten sich vom Acylrest der Stearinsäure sowie von den Acylresten der Mischung der Kokos-Fettsäuren ab, besonders bevorzugt von Linolsäure.

Es wurde gefunden, dass sich Verbindungen der Formel (II), bei denen R3 der Acylrest der Linolsäure ist, durch eine höhere Verträglichkeit mit dem Emulgatorsystem auszeichnen. Dies bedeutet, dass sich diese Substanzen leichter in die Formulierungen einarbeiten lassen. Weiterhin weisen Formulierungen mit Verbindungen der Formel (II), bei denen R3 für den Rest der Linolsäure steht, einen deutlich höheren Pflegeeffekt im Vergleich zu Verbindungen mit gesättigten Fettsäureresten auf. Ethyl- und Methylgruppen sind bevorzugte Alkylgruppen. Ganz besonders bevorzugt sind Methylgruppen.

Verbindungen der Formel (II) sind bereits bekannt. So werden in der EP-A1-13 713 die tensidischen Eigenschaften dieser Verbindungen allgemein beschrieben. Ferner ist aus der DE-A1-44 08 506 der Einsatz einer Verbindung der Formel (II) in Haarfärbemitteln bereits bekannt. Diesen Schriften sind aber keine Hinweise auf die synergistische Steigerung der Pflegewirkung der erfindungsgemäßen Wirkstoffkombinationen zu entnehmen.

Ganz besonders bevorzugte kationisierte Phosphatester der Formel (II) sind die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA, Phospholipid PTC sowie Phospholipid SV vertrieben. Erfindungsgemäß werden die Verbindungen der Formel (II) in Mengen von 0,01 bis 5 Gew.-%, insbesondere in Mengen von 0,05 bis 2 Gew.-%, jeweils bezogen auf das gesamte Mittel, in den beanspruchten Mitteln eingesetzt.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Bevorzugte Oxidationsmittel sind Persulfate, Peroxodisulfate, Chlorite, Hypochlorite und insbesondere Wasserstoffperoxid oder dessen Anlagerungsprodukte an Harnstoff, Melamin sowie Natriumborat.

Eine bevorzugte Ausführungsform der erfindungsgemäßen Verwendung ist daher dadurch gekennzeichnet, dass das Mittel zusätzlich mindestens ein Oxidationsmittel, ausgewählt aus Wasserstoffperoxid und/oder einen seiner Anlagerungsprodukte an organische oder anorganische Verbindungen, enthält. Bevorzugte Anlagerungsprodukte sind die Anlagerungsprodukte von Wasserstoffperoxid an Harnstoff, Melamin sowie Natriumborat. Bevorzugt wird jedoch als Oxidationsmittel Wasserstoffperoxid eingesetzt. Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges H₂O₂), jeweils bezogen auf das anwendungsbereite Mittel.

Erfindungsgemäß kann das Oxidationsfärbemittel zusammen mit einem Katalysator auf das Haar aufgebracht werden, der die Oxidation der Farbstoffvorprodukte, z.B. durch Luftsauerstoff, aktiviert. Solche Katalysatoren sind z. B. Enzyme, Iodide, Chinone oder Metallionen.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht. Bei einer Anwendung von zusätzlichen Oxidationsmitteln wird die eigentliche Färbezubereitung zweckmäßigerweise unmittelbar vor der Anwendung durch Mischung einer erfindungsgemäßen Zubereitung, enthaltend mindestens eine farbverändernde Komponente und mindestens eine Aminosäure, sowie einer Zubereitung, enthaltend das zusätzliche Oxidationsmittel, insbesondere Wasserstoffperoxid, hergestellt.

Weiterhin hat es sich als vorteilhaft erweisen, wenn die Färbemittel mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendi-amintetra(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Seifen, Alkylsulfate, Alkylethersulfate und Ethercarbonsäuren mit 10 bis 18 C-Atomen in Alkylgruppe und bis zu 12 Glykolethergruppen im Molekül. Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine COOH- oder SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet. Weiterhin hat es sich als vorteilhaft erwiesen, wenn die erfindungsgemäßen Färbe- oder Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Als nichtionische Tenside eignen bevorzugt sich Alkylpolyglykoside, insbesondere C₈-C₂₂-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid^{®} S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise Assoziativpolymere mit Fettalkylkette, kationische Polymere, nichtionische Polymere (Vinylpyrrolidinon/Vinylacrylat-Copolymere, Polyvinylpyrrolidinon, Vinylpyrrolidinon/ Vinylacetat-Copolymere, Polyethylenglykole und Polysiloxane); zwitterionische und amphotere Polymere (Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere);
anionische Polymere (Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); haarkonditionierende Verbindungen (Phospholipide, wie Sojalecithin, Ei-Lecitin, Kephaline sowie Silikonöle); zusätzliche Proteinhydrolysate pflanzlicher oder tierischer Herkunft (Elastin-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (Piroctone Olamine, Zink Omadine und Climbazol); Lichtschutzmittel (derivatisierte Benzophenone, Zimtsäure-Derivate und Triazine); Wirkstoffe (Pantolacton, Allantoin, Pyrrolidinoncarbonsäuren und deren Salze sowie Bisabolol); Vitamine, Provitamine und Vitaminvorstufen, insbesondere solche der Gruppen A, B₃, B₅, B₆, C, E, F und H; Pflanzenextrakte (aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Moringa, Hauhechel, Huflattich, Eibisch, Meristem, Ginseng und Ingwer); pflanzliche Öle (Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl, Teebaumöl); Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol / Acrylamid-Copolymere); Perlglanzmittel (Ethylenglykolmonostearat sowie PEG-3-distearat); Treibmittel wie Propan-Butan-Gemische, N₂O, Dimethylether, CO₂ und Luft; Antioxidantien.

Die Auswahl dieser weiteren Stoffe wird der Fachmann gemäß den gewünschten Eigenschaften der Zubereitungen treffen. Die Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die Färbezubereitungen der erfindungsgemäßen Verwendung weisen bevorzugt einen pH-Wert im Bereich von 4 bis 12 aufweist. Im Fall von Oxidationsfärbemitteln findet die Anwendung der Färbemittel in einem schwach alkalischen Milieu statt, bevorzugt bei einem pH-Wert im Bereich von 8,0 bis 10,5. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden ausgewählt aus 2-Aminoethan-1-ol (Monoethanolamin), 2-Amino-2-methylpropan-1-ol, 2-Amino-2-methyl-propan-1,3-diol und Triethanolamin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat, bevorzugt Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin. Schließlich ist ein weiteres bevorzugtes Alkalisierungsmittel Ammoniak.

Die erfindungsgemäßen Mittel können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein oxidatives Färbemittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein zweiter Gegenstand der vorliegenden Erfindung ist daher eine Verpackungseinheit (Kit-of-Parts), welche getrennt voneinander konfektioniert mindestens einen Container (I), enthaltend eine Oxidationsmittelszubereitung (B), die in einem kosmetischen Träger als Oxidationsmittel mindestens Wasserstoffperoxid enthält, und mindestens einen Container (II), enthaltend eine Färbezubereitung (A), die in einem wässrig-kosmetischen Träger mindestens eine farbverändernde Komponente enthält, dadurch gekennzeichnet, dass die Färbezubereitung (A) einen Wirkstoffkomplex enthält, welcher
a) mindestens einen Diester (a) der Formel (I), 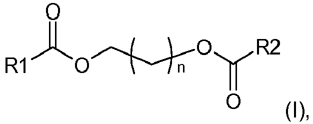 worin
   R1 und R2 jeweils unabhängig voneinander für eine C₇-C₂₃-Alkyl-Gruppe oder C₇-C₂₃-Alkenyl-Gruppe und n für eine Zahl von 1 bis 10 stehen,
b) mindestens ein Proteinhydrolysat (b) mit einem Anteil von Hydroxyprolin von 10 bis 18 Gew.-%, bezogen auf den Proteingesamtgewicht des Proteinhydrolysats, und
c) L-Serin und/oder eines seiner physiologisch verträglichen Salze,
wobei das Verhältnis von Gesamtgewicht an L-Serin und/oder dessen physiologisch verträglichen Salze zu Proteingesamtgewicht des Proteinhydrolysats (b), jeweils bezogen auf das Gesamtgewicht des anwendungsbereiten Mittels, einen Wert von mindestens 1,0 besitzt, enthält, wobei der Wirkstoffkomplex als Proteinhydrolysat mindestens ein Hydrolysat aus Collagen aus "Pisces" enthält. In einer Ausführungsform des zweiten Erfindungsgegenstands enthält die Färbezubereitung (A) als farbverändernde Komponente mindestens ein Oxidationsfarbstoffvorprodukt.

In einer besonders bevorzugten Ausführungsform ist die Verpackungseinheit des zweiten Erfindungsgegenstands dadurch gekennzeichnet, dass sie mindestens eine zusätzliche Komponente, ausgewählt aus der Gruppe, die gebildet wird aus persönlicher Schutzbekleidung, wie Einweghandschuhen, Schürze, Applikationshilfe, wie Kamm, Bürste, Pinsel oder Applicette, und Gebrauchsanleitung enthält. Die Gebrauchsanleitung enthält insbesondere Informationen und Anweisungen für den Verbraucher (m/f) zur Anwendung der Mittel aus den Behältern der Verpackungseinheit in einem Verfahren gemäß dem ersten Erfindungsgegenstand. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der vorliegenden Erfindung ist zur Färbung menschlicher Haare, wobei ein Mittel des ersten Erfindungsgegenstands auf das Haar aufgetragen wird, für einen Zeitraum von 3 bis 45 Minuten, bevorzugt 5 bis 30 Minuten im Haar belassen wird, und das Haar anschließend mit Wasser und/oder einem handelsüblichen Shampoo gespült wird.

Die Auftragungs- und die Einwirktemperatur der Färbezubereitung beträgt Raumtemperatur bis 45°C. Gegebenenfalls kann die Wirkung der Färbezubereitung durch externe Wärmezufuhr, wie beispielsweise mittels einer Wärmehaube, verstärkt werden. Die bevorzugte Einwirkdauer der Färbezubereitung auf die keratinische Faser beträgt 3 bis 45 min, bevorzugt 5 bis 30 min. Nach Beendigung der Einwirkdauer wird das verbliebene Färbemittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet. Die Auftragung der Färbezubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbezubereitung mit einer Applikationshilfe auf die keratinischen Fasern aufzutragen.

In einer Ausführungsform dieses Verfahrens wird das anwendungsbereite Färbemittel durch Vermischen der Färbezubereitung (A) mit der Entwicklerzubereitung (B) der Mehrkomponentenverpackungseinheit des zweiten Erfindungsgegenstands hergestellt, auf die keratinischen Fasern aufgetragen, für eine bestimmte Einwirkzeit im Haar belassen und das Haar anschließend mit Wasser und/oder einem handelsüblichen Shampoo ausgespült.

Ein weiterer Gegenstand der Erfindung ist die kosmetische, nicht-therapeutische Verwendung eines Färbemittels des ersten Erfindungsgegenstands zur Verbesserung der Pflege der Fasern bei der oxidativen Farbveränderung menschlicher Haare.

Ein weiterer Gegenstand der Erfindung ist die kosmetische, nicht-therapeutische Verwendung eines Färbemittels des ersten Erfindungsgegenstands zur Verbesserung der Naßkämmbarkeit der gefärbten Fasern bei der oxidativen Farbveränderung menschlicher Haare.

Ein weiterer Gegenstand der Erfindung ist die kosmetische, nicht-therapeutische Verwendung eines Färbemittels des ersten Erfindungsgegenstands zur Verbesserung der Geschmeidigkeit und des Glanzes bei der oxidativen Farbveränderung menschlicher Haare.

Bezüglich weiterer bevorzugter Ausführungsformen der erfindungsgemäßen Verfahren und Verwendungen gilt mutatis mutandis das zu den erfindungsgemäßen Mitteln Gesagte.

### Beispiele

### Herstellung der Färbecreme

Folgende Färbecremes mit gleicher Farbstoffmischung wurden hergestellt:

| Rohstoffe | **E1** | **V1** |
|---|---|---|
| Farbpulvermischung* | 2,3 | 2,3 |
| Ammonium Carbomer, 1 % | 12,0 | 12,0 |
| Lanette E, Pulver | 0,6 | 0,6 |
| Texapon NSF, 27% | 3,5 | 3,5 |
| Kalium Oleat, 12,5% | 2,4 | 2,4 |
| Cutina GMS SE | 1,6 | 1,6 |
| Cutina AGS | 1,6 | 1,6 |
| Eutanol G | 1,6 | 1,6 |
| Cetearyl Alcohol | 9,6 | 9,6 |
| Ceteareth-20 | 2,4 | 2,4 |
| L-Serin | 0,5 | 0,5 |
| Phospholipid EFA | 0,1 | 0,1 |
| Na₄-EDTA, Pulver, 87% | 0,2 | 0,2 |
| Marine Hydrolyzed Collagen** | 1,0 | -- |
| Ascorbinsäure | 0,1 | 0,2 |
| Natriumsulfit, wasserfrei, 96% | 0,2 | 0,2 |
| Ammoniak, 25% | 6,0 | 6,0 |
| Parfum | qs | qs |
| Wasser | ad 100 | ad 100 |

| | | |
|---|---|---|
| *Farbstoffmischung, enthaltend 59,2 Gew.-% p-Toluylendiaminsulfat, 6,6 Gew.-% 3-Aminophenol, 22,7 Gew.-% Resorcin, 0,8 Gew.-% 2-Amino-4-hydroxyethylaminoanisolsulfat und 10,7 Gew.-% Siliciumdioxid, pyrogen, jeweils bezogen auf das Gesamtgewicht der Mischung. **Marine Hydrolyzed Collagen aus Fischhäuten (Proteingesamtgehalt: ca. 5,5-7,0 Gew.-%; Hydroxyprolin-Gehalt: ca. 0,7-0,9 Gew.-%, jeweils bezogen auf das Gesamtgewicht des Hydrolysats; IMPAG Import GmbH). Rohstoffe: Lanette E, Pulver (INCI-Bezeichnung: Sodium Cetearyl Sulfate; Cognis); Texapon NSF, 27% (INCI-Bezeichnung: Sodium Laureth Sulfate; Cognis); Cutina GMS SE (INCI-Bezeichnung: Glyceryl Stearate; Cognis); Cutina AGS (INCI-Bezeichnung: Glycol Distearate; Cognis); Eutanol G (INCI-Bezeichnung: Octyldodecanol; Cognis); Phospholipid EFA (INCI-Bezeichnung: Linoleamidopropyl PG-dimonium chloride phosphate; Uniqema). | | |

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V1 handelt es sich um eine nicht erfindungsgemäße Vergleichsrezeptur. Die Rezeptur E1 ist ein erfindungsgemäßes Beispiel.

### 1.2. Vermischen mit der Entwicklerdispersion (EW) und Applikation

Jede Färbecreme wurde im Gewichtsverhältnis 1:1 mit einer wie folgt zusammengesetzten Entwicklerdispersion ausgemischt.

| **Rohstoff** | **Gew.-%** |
|---|---|
| Na-benzoat | 0,04 |
| Dipicolinsäure | 0,10 |
| Dinatriumpyrophosphat | 0,19 |
| 1,2-Propandiol | 0,50 |
| HEDP, 60% | 0,25 |
| Paraffinum Liquidum | 0,30 |
| Genamin STAC | 0,20 |
| Cetearyl Alcohol | 3,00 |
| Eumulgin B 2 | 0,70 |
| Wasserstoffperoxid 50 % | 12,2 |
| Kaliumhydroxid, 50 % | 0,19 |
| Wasser | ad 100 |

| | |
|---|---|
| Genamin STAC Trimethylstearylammonium chlorid (ca. 80% Aktivsubstanz; INCI-Bezeichnung: Steartrimonium Chloride) (Clariant) | |

Haarsträhnen (Kerling 6/0) wurden mit 3% Natrium Laurethsulfat gewaschen. Für die Färbung wurde auf den Strähnen von ca. 0,7 g Gewicht die 4-fache Menge der fertigen Anwendungsmischungen appliziert. Nachdem die Strähnen für 30 min bei 32 °C gefärbt wurden, wurden sie für 5 min mit Wasser gespült und an der Luft getrocknet.

### 1.3 Färbeergebnisse und Naßkämmbarkeit

Mit beiden Färbezubereitungen wurde ein satter Braunton erzielt, wobei die mit der erfindungsgemäßen Färbezubereitungen (E1 + EW) bzw. (V1 + EW) behandelten Strähnen deutlich überlegenen Glanz gegenüber den jeweiligen Vergleichsrezepturen zeigten. Vor der Anwendung der Testformulierungen wurden die Strähnen 10x automatisch mit Hartgummikämmen (Hercules Sägemann) gekämmt und die aufzuwendende Arbeit bestimmt. Nach Anwendung der Mischungen V1/EW und E1/EW die Messungen wiederholt. Es wurden jeweils 10 Haarsträhnen behandelt und gemessen. Als Wert für die Kämmarbeit wurde jeweils das arithmetische Mittel gebildet. Folgende Ergebnisse wurden erhalten:

| **Haarbehandlung** | **Kämmarbeit** |
|---|---|
| unbehandelt | -- |
| gefärbt mit FC + V1 (nicht erfindungsgemäß) | +48% |
| gefärbt mit FC + E1 (erfindungsgemäß) | +29% |

Die mit den erfindungsgemäßen Mitteln gefärbten Haarsträhnen zeigten eine deutlich verbesserte Kämmarbeit gegenüber den mit Vergleichsmitteln gefärbten Strähnen. Somit verbessern die erfindungsgemäßen Mittel die keratinische Faseroberfläche signifikant gegenüber entsprechenden Vergleichsmitteln.

## Patentansprüche

1. Mittel zur Farbveränderung von keratinischen Fasern, insbesondere menschlichen Haaren, enthaltend in einem kosmetischen Träger mindestens eine farbverändernde Komponente, **dadurch gekennzeichnet, dass** das Mittel zusätzlich einen Wirkstoffkomplex enthält, welcher
a) mindestens einen Diester (a) der Formel (I), 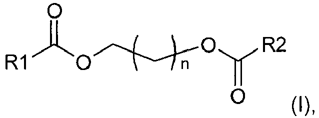 worin
R1 und R2 jeweils unabhängig voneinander für eine C₇-C₂₃-Alkyl-Gruppe oder C₇-C₂₃-Alkenyl-Gruppe und n für eine Zahl von 1 bis 10 stehen,
b) mindestens ein Proteinhydrolysat (b) mit einem Anteil von Hydroxyprolin von 10 bis 18 Gew.-%, bezogen auf den Proteingesamtgewicht des Proteinhydrolysats, und
c) L-Serin und/oder eines seiner physiologisch verträglichen Salze,
wobei das Verhältnis von Gesamtgewicht an L-Serin und/oder dessen physiologisch verträglichen Salze zu Proteingesamtgewicht des Proteinhydrolysats (b), jeweils bezogen auf das Gesamtgewicht des anwendungsbereiten Mittels, einen Wert von mindestens 1,0 besitzt, wobei der Wirkstoffkomplex als Proteinhydrolysat mindestens ein Hydrolysat aus Collagen aus "*Pisces*" enthält,
umfasst.

2. Mittel nach Anspruch 1, **dadurch gekennzeichnet, dass** der Wirkstoffkomplex als Diester der Formel (I) mindestens eine Verbindung enthält, bei der n für die Zahl 1 und R1 und R2 jeweils für eine C₁₇-Alkyl-Gruppe, eine C₁₁-Alkyl-Gruppe oder eine C₁₅-Alkyl-Gruppe stehen.

3. Mittel einem der Ansprüche 1 bis 2, **dadurch gekennzeichnet, dass** das Mittel einen Wirkstoffkomplex enthält, welcher den oder die Diester (a) der Formel (I) zu 0,01 bis 10,0 Gew.-%, bevorzugt zu 0,1 bis 5,0 Gew.-%, ein oder mehrere Proteinhydrolysate (b) mit einem Proteingesamtgehalt von 0,001 bis 0,5 Gew.-%, bevorzugt von 0,005 bis 0,2 Gew.-%, und L-Serin und/oder dessen physiologisch verträglichen Salze von 0,05 bis 3,0 Gew.-%, bevorzugt zu 0,1 bis 2,0 Gew.-%, jeweils bezogen auf das Gesamtgewicht des anwendungsbereiten Mittels, enthält.

4. Mittel nach einem der Ansprüche 1 bis 3, **dadurch gekennzeichnet, dass** das Mittel als farbverändernde Komponente mindestens ein Oxidationsfarbstoffvorprodukt und/oder mindestens einen direktziehenden Farbstoff und/oder mindestens ein Aufhellmittel enthält.

5. Mittel nach Anspruch 4, **dadurch gekennzeichnet, dass** es als farbverändernde Komponente mindestens ein Oxidationsfarbstoffvorprodukt enthält.

6. Mittel nach einem der Ansprüche 1 bis 5, **dadurch gekennzeichnet, dass** das Mittel zusätzlich mindestens einen Pflegestoff, ausgewählt aus kationisierten Phosphatestern, enthält.

7. Mittel nach einem der Ansprüche 1 bis 6, **dadurch gekennzeichnet, dass** das Mittel zusätzlich ein Oxidationsmittel, ausgewählt aus Wasserstoffperoxid sowie seinen festen Anlagerungsprodukten an organische und anorganische Verbindungen, enthält.

8. Verwendung eines Mittels gemäß einem der Ansprüche 1 bis 7 zur Verbesserung der Naßkämmbarkeit, der Geschmeidigkeit und des Glanzes bei der oxidativen Farbveränderung menschlicher Haare.

9. Mehrkomponentenverpackungseinheit (Kit-of-Parts), enthaltend mindestens zwei getrennt konfektionierte Container, umfassend mindestens einen Container (I), enthaltend eine Oxidationsmittelszubereitung (B), die in einem kosmetischen Träger als Oxidationsmittel mindestens Wasserstoffperoxid enthält, und mindestens einen Container (II), enthaltend eine Färbezubereitung (A), die in einem wässrig-kosmetischen Träger mindestens eine farbverändernde Komponente enthält, **dadurch gekennzeichnet, dass** die Färbezubereitung (A) einen Wirkstoffkomplex enthält, welcher
a) mindestens einen Diester (a) der Formel (I), 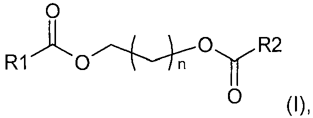 worin
R1 und R2 jeweils unabhängig voneinander für eine C₇-C₂₃-Alkyl-Gruppe oder C₇-C₂₃-Alkenyl-Gruppe und n für eine Zahl von 1 bis 10 stehen,
b) mindestens ein Proteinhydrolysat (b) mit einem Anteil von Hydroxyprolin von 10 bis 18 Gew.-%, bezogen auf den Proteingesamtgewicht des Proteinhydrolysats, und
c) L-Serin und/oder eines seiner physiologisch verträglichen Salze umfasst,
wobei das Verhältnis von Gesamtgewicht an L-Serin und/oder dessen physiologisch verträglichen Salze zu Proteingesamtgewicht des Proteinhydrolysats (b), jeweils bezogen auf das Gesamtgewicht des anwendungsbereiten Mittels, einen Wert von mindestens 1,0 besitzt, wobei der Wirkstoffkomplex als Proteinhydrolysat mindestens ein Hydrolysat aus Collagen aus "*Pisces*" enthält.

## Claims

1. An agent for changing the color of keratin fibers, in particular human hair, containing, in a cosmetic carrier, at least one color-changing component, **characterized in that** the agent additionally contains an active ingredient complex that contains
a) at least one diester (a) of formula (I), 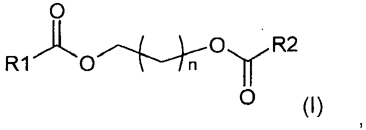 where
R1 and R2, each independently of one another, represent a C₇-C₂₃ alkyl group or a C₇-C₂₃ alkenyl group, and n represents a number from 1 to 10,
b) at least one protein hydrolyzate (b) having a hydroxyproline content of from 10 to 18 wt.%, based on the total protein weight of the protein hydrolyzate, and
c) L-serine and/or one of the physiologically acceptable salts thereof,
the ratio of the total weight of L-serine and/or the physiologically acceptable salts thereof to the total protein weight of the protein hydrolyzate (b), in each cased based on the total weight of the ready-to-apply agent, having a value of at least 1.0, the active ingredient complex containing at least one hydrolyzate consisting of "*pisces*" collagen as the protein hydrolyzate.

2. The agent according to claim 1, **characterized in that** the active ingredient complex contains at least one compound as the diester of formula (I), in which compound n represents the number 1 and R1 and R2 each represent a C₁₇ alkyl group, a C₁₁ alkyl group or a C₁₅ alkyl group.

3. The agent according to one of claims 1 or 2, **characterized in that** the agent contains an active ingredient complex that contains the diester(s) (a) of formula (I) at 0.01 to 10.0 wt.%, preferably at 0.1 to 5.0 wt.%, one or more protein hydrolyzates (b) having a total protein content of from 0.001 to 0.5 wt.%, preferably from 0.005 to 0.2 wt.%, and L-serine and/or the physiologically acceptable salts thereof of 0.05 to 3.0 wt.%, preferably at 0.1 to 2.0 wt.%, in each case based on the total weight of the ready-to-apply agent.

4. The agent according to one of claims 1 to 3, **characterized in that** the agent contains at least one oxidation dye precursor and/or at least one substantive dye and/or at least one lightening agent as the color-changing component.

5. The agent according to claim 4, **characterized in that** it contains at least one oxidation dye precursor as the color-changing component.

6. The agent according to one of claims 1 to 5, **characterized in that** the agent additionally contains a nourishing substance selected from cationized phosphate esters.

7. The agent according to one of claims 1 to 6, **characterized in that** the agent additionally contains an oxidizing agent selected from hydrogen peroxide and solid addition products thereof of organic and inorganic compounds.

8. The use of an agent according to one of claims 1 to 7 for improving the ability to be wet-combed, the smoothness and the shine during oxidative color-changing of human hair.

9. A kit-of-parts containing at least two separately packaged containers, comprising at least one container (I) containing an oxidizing agent preparation (B) that contains, in a cosmetic carrier, at least hydrogen peroxide as the oxidizing agent, and at least one container (II) containing a color preparation (A) that contains at least one color-changing component in an aqueous cosmetic carrier, **characterized in that** the color preparation (A) contains an active ingredient complex that contains
a) at least one diester (a) of formula (I), 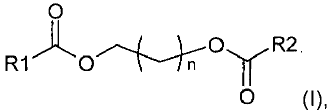 where
R1 and R2, each independently of one another, represent a C₇-C₂₃ alkyl group or C₇-C₂₃ alkenyl group, and n represents a number from 1 to 10,
b) at least one protein hydrolyzate (B) having a hydroxyproline content of from 10 to 18 wt.%, based on the total protein weight of the protein hydrolyzate, and
c) L-serine and/or one of the physiologically acceptable salts thereof,
the ratio of the total weight of L-serine and/or the physiologically acceptable salts thereof to the total protein weight of the protein hydrolyzate (b), in each cased based on the total weight of the ready-to-apply agent, having a value of at least 1.0, the active ingredient complex containing at least one hydrolyzate consisting of "*pisces*" collagen as the protein hydrolyzate.

## Revendications

1. Agent pour la modification de la couleur de fibres kératiniques, notamment de cheveux humains, contenant dans un support cosmétique au moins un composant pour la modification de la couleur, **caractérisé en ce que** l'agent contient en outre un complexe de principes actifs, lequel comprend
a) au moins un diester (a) de la formule (I), 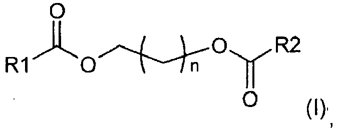 dans laquelle
R1 et R2 représentent respectivement indépendamment l'un de l'autre un groupe alkyle en C₇-C₂₃ ou un groupe alcényle en C₇-C₂₃ et n représente un chiffre de 1 à 10,
b) au moins un hydrolysat de protéine (b) avec une part d'hydroxyproline de 10 à 18 % en poids rapporté au poids total en protéine de l'hydrolysat de protéine, et
c) de la sérine-L et/ou l'un de ses sels physiologiquement compatibles
dans lequel le rapport du poids total sur sérine-L et/ou son sel physiologiquement compatible sur le poids total en protéine de l'hydrolysat de protéine (b), respectivement rapporté au poids total de l'agent prêt à l'emploi, possède une valeur d'au moins 1,0,
dans lequel le complexe de principes actifs contient au moins un hydrolysat de collagène de « *Pisces* » (poisson) en tant qu'hydrolysat de protéine.

2. Agent selon la revendication 1, **caractérisé en ce que** le complexe de principes actif contient au moins, en tant diester de la formule (I), un composé dans lequel n représente le chiffre 1 et R1 et R2 représentent respectivement un groupe alkyle en C₁₇, un groupe alkyle en C₁₁ ou un groupe alkyle en C₁₅.

3. Agent selon l'une quelconque des revendications 1 à 2, **caractérisé en ce que** l'agent contient un complexe de principes actifs, lequel contient de 0,01 à 10,0 % en poids, préférablement de 0,1 à 5,0 % en poids en diester(s) (a) de la formule (I), un ou plusieurs hydrolysats de protéine (b) avec une teneur totale en protéine de 0,001 à 0,5 % en poids, préférablement de 0,005 à 0,2 % en poids et de la sérine-L et/ou son sel physiologiquement compatible de 0,05 à 3,0 % en poids, préférablement de 0,1 à 2,0 % en poids, respectivement rapporté au poids total de l'agent prêt à l'emploi.

4. Agent selon l'une quelconque des revendications 1 à 3, **caractérisé en ce que** l'agent contient, en tant que composant de modification de la couleur, au moins un précurseur de colorant par oxydation et/ou au moins un colorant à action directe et/ou au moins un agent éclaircissant.

5. Agent selon la revendication 4, **caractérisé en ce qu'**il contient au moins un précurseur de colorant par oxydation en tant que composant de modification de la couleur.

6. Agent selon l'une quelconque des revendications 1 à 5, **caractérisé en ce que** l'agent contient en outre au moins un nutriment, sélectionné parmi les esters de phosphate cationisés.

7. Agent selon l'une quelconque des revendications 1 à 6, **caractérisé en ce que** l'agent contient en outre un agent d'oxydation, sélectionné parmi le peroxyde d'hydrogène ainsi que ses produits d'addition solides sur des composés organiques et inorganiques.

8. Utilisation d'un agent selon l'une quelconque des revendications 1 à 7 pour l'amélioration des possibilités de se peigner avec les cheveux humides, de la souplesse et de l'éclat lors de la modification par oxydation de la couleur de cheveux humains.

9. Unité de conditionnement à composants multiples (kit-of-parts), contenant au moins deux conteneurs confectionnés séparément, comprenant au moins un conteneur (I), contenant une préparation d'agent d'oxydation (B), qui contient au moins du peroxyde d'hydrogène en tant qu'agent d'oxydation dans un support cosmétique, et au moins un conteneur (II), contenant un préparation de coloration (A), qui contient au moins un composant de modification de la couleur dans un support aqueux-cosmétique, **caractérisé en ce que** la préparation de coloration (A) contient un complexe de principes actifs, lequel comprend
a) au moins un diester (a) de la formule (I), 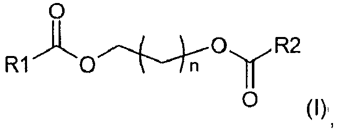 dans laquelle
R1 et R2 représentent respectivement indépendamment l'un de l'autre un groupe alkyle en C₇-C₂₃ ou un groupe alcényle en C₇-C₂₃ et n représente un chiffre de 1 à 10,
b) au moins un hydrolysat de protéine (b) avec une part d'hydroxyproline de 10 à 18 % en poids rapporté au poids total en protéine de l'hydrolysat de protéine, et
c) de la sérine-L et/ou l'un de ses sels physiologiquement compatibles
dans lequel le rapport du poids total sur sérine-L et/ou son sel physiologiquement compatible sur le poids total en protéine de l'hydrolysat de protéine (b), respectivement rapporté au poids total de l'agent prêt à l'emploi, possède une valeur d'au moins 1,0,
dans lequel le complexe de principes actifs contient au moins un hydrolysat de collagène de « *Pisces* » (poisson) en tant qu'hydrolysat de protéine.
